(19) Europäisches Patentamt
European Patent Office
Office européen des brevets

(11) Numéro de publication: **0 199 618**
**A1**

(12) # DEMANDE DE BREVET EUROPEEN

(21) Numéro de dépôt: **86400603.6**

(22) Date de dépôt: **21.03.86**

(51) Int. Cl.[4]: **C 07 D 471/14**
//(C07D471/14, 221:00, 221:00, 209:00)

(30) Priorité: **22.03.85 FR 8504870**

(43) Date de publication de la demande:
**29.10.86 Bulletin 86/44**

(84) Etats contractants désignés:
**AT BE CH DE FR GB IT LI LU NL SE**

(71) Demandeur: **SANOFI**
**40, Avenue George V**
**F-75008 Paris(FR)**

(71) Demandeur: **CENTRE NATIONAL DE LA RECHERCHE SCIENTIFIQUE (CNRS)**
**13 Quai Anatole France**
**F-75700 Paris(FR)**

(72) Inventeur: **Bisagni, Emile**
**16 rue Bossuet**
**F-91400 Orsay(FR)**

(72) Inventeur: **Chi Hung, Nguyen**
**7 allée des Amonts**
**F-91940 Les Ulis(FR)**

(72) Inventeur: **Bras, Jean-Pierre**
**34 rue de Rémusat**
**F-31000 Toulouse(FR)**

(74) Mandataire: **Polus, Camille et al,**
**c/o Cabinet Lavoix 2, Place d'Estienne d'Orves**
**F-75441 Paris Cedex 09(FR)**

(54) **Nouvelles 5-H pyrido (3',4':4,5) pyrrolo (3,2-c) pyridones, leur procédé de préparation et leur application en tant qu'intermédiaires de synthèse.**

(57) La présente invention est relative à de nouveaux dérivés de la 5-H pyrido (3', 4' : 4,5) pyrrolo (3,2-c) pyridone-1 de formule

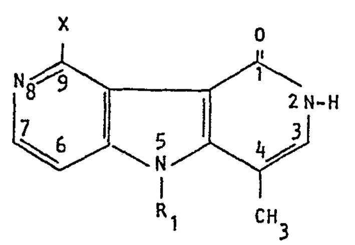

dans laquelle X représente l'hydrogène ou le chlore, $R_1$ représente l'hydrogène, un groupe alcoyle inférieur ou benzyle, leur procédé de préparation et leur application en tant qu'intermédiaires de synthèse dans la préparation de nouveaux composés utilisables dans l'industrie pharmaceutique.

L'invention est relative à de nouveaux dérivés de la 5-H pyrido (3', 4' : 4,5) pyrrolo (3,2-c) pyridones-1 à un procédé pour les préparer et à leur utilisation en tant qu'intermédiaires de synthèse dans la préparation de composés utiles dans l'industrie pharmaceutique.

Les nouveaux dérivés de l'invention répondent à la formule suivante

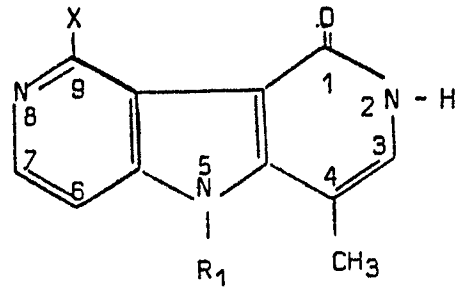

(I)

dans laquelle X représente l'hydrogène ou le chlore, $R_1$ représente l'hydrogène, un groupe alcoyle inférieur ou benzyle.

L'invention concerne aussi les formes tautomères lorsqu'elles existent.

Par radical alcoyle inférieur, on entend une chaîne hydrocarbonée saturée en $C_1$ - $C_4$, linéaire ou ramifiée.

Ces dérivés sont des intermédiaires dans la préparation de nouveaux composés de formule

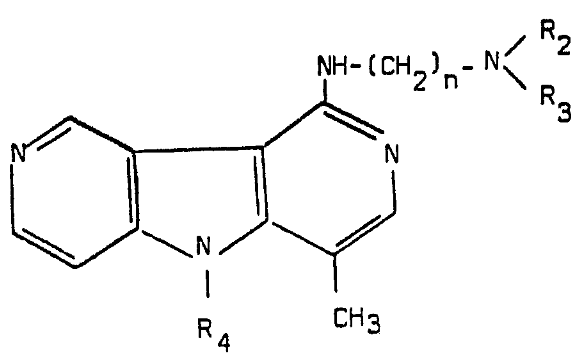

(A)

dans laquelle n représente un nombre entier de 2 à 4, $R_2$ et $R_3$ sont chacun indépendamment l'un de l'autre l'hydrogène, un groupe alcoyle inférieur ou hydroxyalcoyle, et $R_4$ représente l'hydrogène ou un radical alcoyle inférieur.

Les nouveaux composés de formule A sont doués de propriétés antitumorales intéressantes et ils sont utiles en thérapeutique. Ils font l'objet de la demande française 85.04872 déposée ce même jour.

L'invention a également pour objet un procédé de préparation des composés de formule (I) ci-dessus caractérisée en ce que :

a) on oxyde une (hydroxy-1 éthyl-1)-2 chloro-4 1-H pyrrolo (3,2-c) pyridine substituée en position 1, de formule

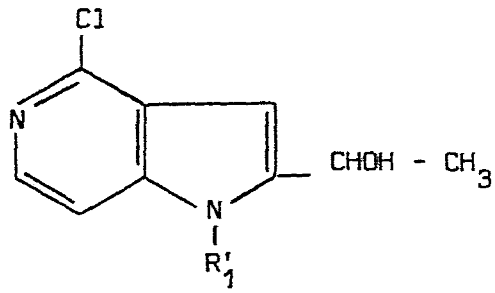

(II)

dans laquelle $R'_1$ est un groupement alcoyle inférieur ou benzyle, en acétyl-2 pyrrolo-pyridine correspondante de formule

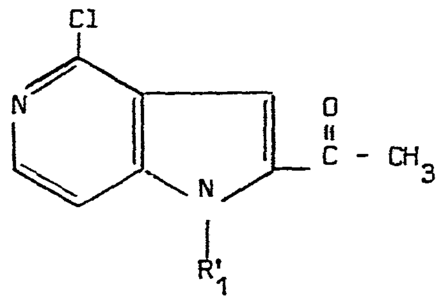

(III)

dans laquelle $R'_1$ est comme dans la formule II,

b) on transforme par une réaction de Wittig-Horner le composé de formule III en (pyrrolo-pyridyl-2)-3 crotonate correspondant de formule

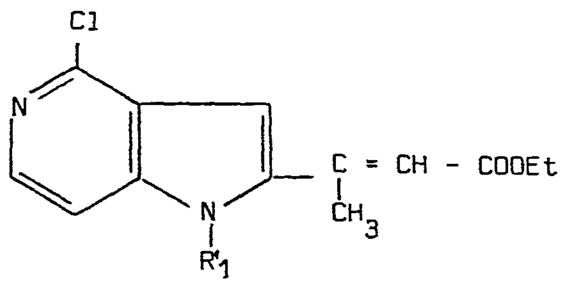

(IV)

dans laquelle $R'_1$ est comme dans la formule II,

c) on saponifie l'ester obtenu de formule IV pour obtenir l'acide [chloro-4 1-H pyrrolo (3,2-c) pyridyl-2]-3 crotonique correspondant de formule

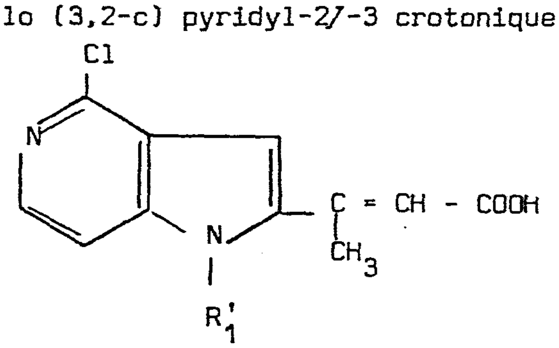

(V)

dans laquelle $R'_1$ est comme dans la formule II.

4°) on transforme le composé de formule (V) en acide de formule

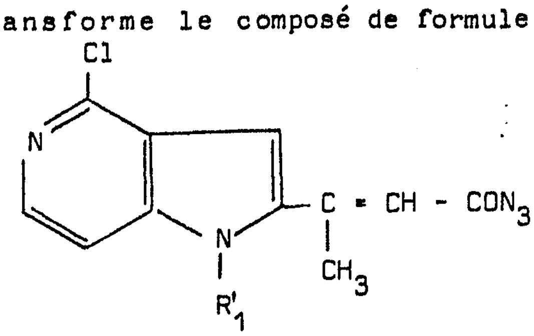

(VI)

dans laquelle R'$_1$ est comme dans la formule II,

5°) on cyclise l'acide de formule (VI) pour obtenir un composé de formule I, X étant le chlore

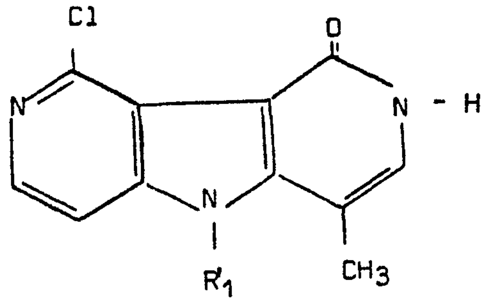

(I)

dans laquelle R'$_1$ représente un groupement alcoyle ou benzyle, puis le cas échéant

6°) par hydrogénation, on obtient le composé de formule I, X représentant l'hydrogène

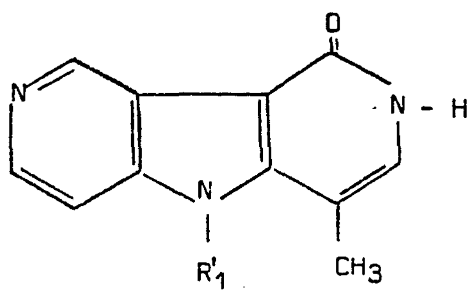

(I)

dans laquelle R'$_1$ représente un groupement alcoyle inférieur ou benzyle.

Ce composé peut être utilisé directement pour obtenir les composés de formule (A) dans lesquels R$_4$ représente un groupe alcoyle inférieur. Il sera débenzylé si l'on veut obtenir l'intermédiaire de formule I dans laquelle X et R$_1$ représentent chacun un hydrogène :

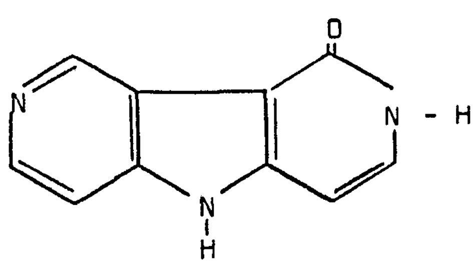

(I)

destiné à préparer les composés de formule A dans lesquels $R_4$ représente l'hydrogène.

Les alcools de formule (II) point de départ de la préparation, sont obtenus à partir des chloro-4 1-H pyrrolo (3,2-c) pyridines substituées en position 1 selon la méthode décrite par E. BISAGNI et Coll. (Tétrahedron, 39, 1777-1781, (1983)) en utilisant toutefois, comme solvant, dans le cas où le substituant est un groupe benzyle, l'éther diéthylique.

L'oxydation des alcools en dérivés acétylés de formule (III) est effectuée par le bioxyde de manganèse au sein d'un solvant chloré porté au reflux.

La transformation en ester crotonique de formule (IV) est obtenue en faisant réagir sur le dérivé de formule (III), en solution dans un solvant inerte tel que le diméthoxy-1,2 éthane, à une température comprise entre 0°C et 90°C, le diéthyl phosphonoacétate d'éthyle préalablement sodé par l'hydrure de sodium. On obtient un mélange cis-trans de l'ester éthylique de l'acide crotonique de formule (IV).

Cet ester est directement saponifié en acide [chloro-4 1-H pyrrolo (3,2-c) pyridyl-2]-3 crotonique de formule (V).

Cet acide est transformé en anhydride mixte, en solution acétonique, par action du chlorocarbonate d'éthyle à basse température en présence d'une amine aliphatique tertiaire, puis en azide de formule (VI) par action de l'azoture de sodium en solution aqueuse à basse température.

L'azide en solution dans un solvant inerte tel que le diphényléther est cyclisé par chauffage à haute température en présence d'une amine aliphatique tertiaire de point d'ébullition élevé telle que la tributylamine. On obtient ainsi les composés de formule (I) dans lesquels X représente le chlore et $R_1$ est un groupe alcoyle inférieur ou benzyle.

Par hydrogénation, à température et pression ambiantes, en présence d'un catalyseur tel que le charbon palladié, on obtient les composés de formule (I) dans lesquels X représente l'hydrogène et $R_1$ est toujours un groupe alcoyle inférieur ou benzyle.

Pour obtenir les composés de formule (I) dans lesquels $R_1$ représente l'hydrogène, on soumet à une débenzylation les composés dans lesquels $R_1$ est un groupe benzyle : cette opération est réalisée par l'action du sodium dans l'amoniac liquide.

Les exemples suivants sont donnés à titre d'exemple non limitatif de la présente invention.

EXEMPLE 1 : chloro-9 diméthyl-4,5 2-H 5-H pyrido (3', 4' : 4,5) pyrrolo (3,2-c) pyridone-1

(X = Cl - $R_1$ = $CH_3$) - dérivé n° 1

X a) méthyl-1 acétyl-2 chloro-4 1-H pyrrolo (3,2-c) pyridine (dérivé de formule (III))

La chloro-4 méthyl-1 (hydroxy-1 éthyl)-2 1-H pyrrolo (3,2-c) pyridine (1 g) est dissoute dans 30 ml de chloroforme. On ajoute 8 g de bioxyde de manganèse, puis, sous agitation, le milieu réactionnel est chauffé à reflux pendant 22 heures. Après refroidissement et filtration, la solution est évaporée sous vide et le résidu recristallisé dans l'éthanol.

On obtient 0,780 g du produit recherché

rendement : 78 p. 100 - F = 215-216°C.

Calc. pour $C_{10}H_9ClN_2O$ = C, 57,55 ; H, 4,32 ; N, 13,43 ; Cl, 17,03 ;

Trouvé : C, 57,42 ; H, 4,29 ; N, 13,31 ; Cl, 16,73

X b) [ méthyl-1 chloro-4 1-H pyrrolo (3,2-c) pyridyl-2 ] -3 crotonate d'éthyle (dérivé de formule (IV) - mélange cis-trans)

A une suspension d'hydrure de sodium (1,34 g à 0,50 p. 100 dans l'huile, 28 mmoles) dans 100 ml de diméthoxy-1,2 éthane, on ajoute 6,3 g (28 mmoles) de diéthylphosphonoacétate d'éthyle en solution dans 90 ml de diméthoxy-1,2 éthane distillé sur hydrure de calcium. Après 3 heures à 0°C sous agitation, 4,17 g de la cétone précédemment obtenue de formule (III) en suspension dans 100 ml de diméthoxy-1,2 éthane sont ajoutés progressivement. Le mélange réactionnel est laissé pendant 2 heures à 0°C, 18 heures à la température ambiante, puis chauffé à reflux pendant 4 heures. Après évaporation du solvant, le mélange est versé dans l'eau, extrait au chlorure de méthylène et par évaporation de ce dernier on recueille le produit recherché sous forme d'huile .

X c) Acide (méthyl-1 chloro-4 1-H pyrrolo (3,2-c) pyridyl-2)-3 crotonique (dérivé de formule (V) mélange cis-trans)

L'ester obtenu dans l'étape précédente est dissous dans 50 ml d'éthanol refroidi à 0°C. Puis une solution d'hydroxyde de potassium (4,57 g, 4 équivalents)dans 5 ml d'eau et 50 ml d'alcool refroidie à 0°C est ajoutée lentement. Après 48 heures à 0°C, l'éthanol est évaporé sous pression réduite en évitant de chauffer et le résidu, dissous dans 200 ml d'eau glacée, est extrait

trois fois avec 100 ml de chlorure de méthylène. La phase aqueuse est acidifiée par l'acide acétique agitée 1 heure à 0°C. Par filtration, on recueille 4,7 g du composé recherché.

Rendement : 93,8 p. 100 - F = 244°C. - 246°C. avec décomposition

Calc pour $C_{12}H_{11}ClN_2O_2$ : C, 57,50 ; H, 4,42 ; N, 11,17 ; Cl, 14,14

Trouvé : C, 57,16 ; H, 4,14 ; N, 11,01 ; Cl, 14,16 ;

RMN : $H_1$ $((CD_3)_2SO)$ ; $\delta$ : 3,42 (d, 3H, $CH_3$-C=) ; 3,65 (s, 3H, N-$CH_3$) ; 5,31 et 5,48 (2s, 1H -C = CH- mélange cis-trans) ; 6,44 (s, 1H, H-3) ; 7,35 (d, 1H, $H_7$, $J_{6-7}$ = 5,5 Hz) ; 7,83 (d, 1H, H-6)

X d) azide dérivé de formule (VI)

La solution de l'acide précédemment obtenue (4,7 g 18 moles) dans 30 ml d'acétone et 2,9 ml de triéthylamine est refroidie à -10°C et on ajoute lentement une solution de 2 ml de chlorocarbonate d'éthyle dans 30 ml d'acétone, en maintenant la température à -10°C. Après 1 heure d'agitation, à la même température, l'azoture de sodium (1,77 g) dissous dans le minimum d'eau est ajouté progressivement et le mélange, maintenu en dessous de -5°C, est agité pendant 2 heures. L'acétone est évaporée sous pression réduite en maintenant le bain-marie en dessous de 10°C, on reprend le résidu dans 200 ml d'eau et on extrait immédiatement au chlorure de méthylène.

La phase organique est séchée sur sulfate de sodium, puis filtrée. L'évaporation à sec sous pression réduite à la température ordinaire fournit le dérivé recherché.

X e) Diméthyl-4,5 chloro-9 2-H 5-H pyrido (3', 4' : 4,5) pyrrolo (3,2-c) pyridone-1 (dérivé de formule (I))

L'azide obtenu dans l'étape précédente est dissous dans le diphényléther chauffé à 35°C - 40°C et ajouté par petites portions à un mélange de 80 ml de diphényléther et de 4,4 ml de tributylamine, chauffé et maintenu à 230°C - 235°C sous bonne agitation.

A la fin de l'addition, le mélange est maintenu pendant 10 minutes à 230°C - 240°C, puis refroidi et additionné de 400 ml d'éther de pétrole.

Le solide formé est essoré, lavé à l'éther de pétrole, puis repris dans 30 ml d'éthanol bouillant.

Le produit insoluble à froid est filtré et séché pour donner des aiguilles incolores.

On recueille ainsi 2,2 g du produit recherché avec un rendement de 47 p. 100 par rapport à l'acide de formule (V).

Fusion : infusible à 260°C.

Calc. pour $C_{12}H_{10}$ Cl $N_3O$ : C, 58, 19 ; H, 4,07 ; N, 16,96 ; Cl, 14,31 ;

Trouvé : C, 57,95 ; H, 3,86 ; N, 16,75 ; Cl. 14,34

RMN : $H_1$ (($CD_3)_2SO$) ; δ : 1,56 (s, 3H, $CH_3$-4) ; 3,13 (s, 3H, N-$CH_3$) ; 6,24 (s, 1H, H-3) ; 6,73 (d, 1H, H-6, $J_{6-7}$= 6 Hz ; 7,26 (d, 1H, H-7) ; 10,36 (s, 1H, N-H).

<u>EXEMPLE 2</u> : chloro-9 méthyl-4 benzyl-5 2-H 5-H pyrido (3', 4' : 4,5) pyrrolo (3,2-c) pyridone-1 (X = Cl, $R_1$ = benzyle) dérivé n° 2

X a) Benzyl-1 acétyl-2 chloro-4 1-H pyrrolo (3,2-c) pyridine (dérivé de formule (III))

On dissout 11,3 g de la benzyl-1 (hydroxy-1 éthyl)-2 chloro-4 1-H pyrrolo (3,2-c) pyridine dans 600 ml de chloroforme bouillant. On ajoute progressivement 120 g de bioxyde de manganèse et on chauffe le mélange à reflux pendant 18 heures sous agitation. Après addition de 40 g bioxyde de manganèse et 2 heures de chauffage à reflux jusqu'à disparition de l'alcool, le bioxyde de manganèse est filtré, lavé avec le chloroforme chaud et le solvant est évaporé. Par recristallisation du résidu dans l'éthanol, on obtient 8,6 g du dérivé recherché.

Rendement : 76,6 p. 100 - F = 136°C - 138°C.

Calc. pour $C_{16}H_{13}$Cl $NO_2$ : C, 67,49 ; H, 4,6 ; N, 9,84 ; Cl, 12,45

Trouvé : C, 67,21 ; H, 4,39 ; N, 10,12 ; Cl, 12,75

RMN $H_1$ (($CD_3)_2SO$), δ : 2,68 (s, 3H, -CO-$CH_3$) ; 5,88 (s, 2H, -$CH_2$-) ; 7,05 - 7,32 (m, 5H, -$C_6H_5$) ; 7,66 (q, 1H, H-7, $J_{3-7}$ = 0,8Hz) ; 7,76 (d, 1H, H-3) ; 8,17 (d, 1H, H-6)

X b) acide (benzyl-1 chloro-4 1-H pyrrolo (3,2-c) pyridyl-2) crotonique dérivé de formule (V)).

De même que dans l'exemple 1, à une suspension d'hydrure de sodium (2,36 g à 50 p. 100 dans l'huile) dans 50 ml de diméthoxyéthane, on ajoute 12,12 g (54 mmoles) de diéthylphosphonoacétate d'éthyle en solution dans 90 ml de diméthoxyéthane et 7 g (25 mmoles) du composé de formule (III) précédemment obtenu en solution dans 160 ml de diméthoxyéthane, et le mélange réactionnel est laissé 48 heures à la température ambiante.

Après évaporation du solvant, décomposition par l'éthanol, extraction au chlorure de méthylène, saponification du résidu de l'évaporation par l'hydroxyde de potassium en excès (5,6 g) en milieu hydroalcoolique et acidification par l'acide acétique, le solide obtenu est filtré et séché pour donner 7,9 g (98 p. 100) de l'acide recherché, en mélange cis-trans.

F = 170°C.

Calc. pour $C_{18}H_{15}$ Cl $N_2O_2$ : C, 66,16 ; H, 4,63 ; N, 8,57 ; Cl, 10,85.

Trouvé : C, 65,86 ; H, 4,81 ; N, 8,35 ; Cl, 11,08

X c) chloro-9 méthyl-4 benzyl-5 2-H 5-H pyrido (3', 4' : 4,5) pyrrolo (3,2-c) pyridone-1 (dérivé de formule (I)).

Ce composé a été obtenu, par l'intermédiaire de l'azide selon la méthode écrite dans l'exemple 1.

En partant de 7,9 g de l'acide de formule (V) on obtient, après purification par ébullition dans le dioxanne, 2,8 g de microcristaux incolores du dérivé de formule (I).

Rendement : 35,7 p. 100 - F = infusible à 260°C.

Calc. pour $C_{18}H_{14}$Cl $N_3O$ : C, 66,77 ; H, 4,36 ; N, 12,98 ; Cl, 10,95 ;

Trouvé : C, 66,52 ; H, 4,57 ; N, 12,69 ; Cl, 10,69.

<u>EXEMPLE 3</u> : Diméthyl-4,5 2-H 5-H pyrido (3', 4' : 4,5) pyrrolo (3,2-c) pyridone-1 (X = H, $R_1$ = $CH_3$) dérivé n° 3.

Le dérivé n° 1, le chloro-9 diméthyl-4,5 2-H 5-H pyrido (3', 4' :4,5) pyrrolo (3,2-c) pyridone-1 (X = Cl, $R_1$ = $CH_3$) (10 mmoles) est placé dans un tricol de 1 litre contenant 500 ml d'éthanol, 7,5 ml de triéthylamine et 700 mg de charbon palladié à 10 p. 100, puis agité sous atmosphère d'hydrogène à la pression normale et à la température ambiante pendant environ 2 heures. Le catalyseur est filtré, lavé plusieurs fois à l'éthanol bouillant et le solvant évaporé. Le résidu est repris dans 200 ml d'une solution d'hydroxyde de sodium N filtré à froid et recristallisé dans l'éthanol pour donner des micro cristaux incolores du composé recherché hydraté.

Rendement 88 p. 100 - F = infusible à 260°C.

Calc. pour $C_{12}H_{11}N_3O$, $H_2O$ = C, 62,34 ; H, 5,63 ; N, 18,18 ;

Trouvé : C, 62,37 ; H, 5,55 ; H, 18,07.

RMN : $H_1$ ($(CD_3)_2SO$) ; $\delta$ : 2,57 (s, 3H, $CH_3$-4) ; 4,14 (s, 3H, N-$CH_3$) ; 7,23 (s, 1H, H-3) ; 7,7 (d, 1H, H-6, $J_{6-7}$ = 6Hz) ; 8,49 (d, 1H, H-7 ; 9,21 (s, 1H, NH-2) ; 9,36 (s, 1H, H-9)

EXEMPLE 4 : méthyl-4 benzyl-5 2-H 5-H pyrido (3', 4' : 4,5) pyrrolo (3,2-c) pyridone-1 (X = H, $R_1$ = benzyle) dérivé n° 4

Ce composé a été préparé à partir du dérivé n° 2, la chloro-9 méthyl-4 benzyl-5 2-H 5-H pyrido (3', 4' : 4,5) pyrrolo (3,2-c) pyridone-1, par le même mode opératoire que dans l'exemple 3.

Rendement : 92 p. 100 F = infusible - 260°C.

Calc. pour $C_{18}H_{15}N_3O$ ; 1,5 $H_2O$ : C, 68,35 ; H, 5,7 ; N, 13,28

Trouvé : C, 68,13 ; H, 6,24 ; N, 12,75

RMN : $H_1$ $((CD_3)_2SO)$ ; $\delta$ : 2,3 (s, 3H, $CH_3$) ; 5,88 (s, 2H, $CH_2$) ; 6,88-7,3 (m, 5H, - $C_6H_5$) ; 7,63 (q, 1H, H-6, $J_{6-7}$ = 5,6 Hz, $J_{6-9}$ = 0,9Hz) ; 8,43 (d, 1H, H-7) ; 9,41 (d, 1H, H-9) ; 11,43 (s, 1H, NH-2)

EXEMPLE 5 : Méthyl-4 2-H 5-H pyrido (3', 4' : 4,5) pyrrolo (3,2-c) pyridone-1 (X = H, $R_1$ = H) dérivé n° 5.

Dans un tricol de 500 ml plongé dans un bain réfrigérant à -70°C surmonté d'un réfrigérant à acétone + carboglace, muni d'un agitateur magnétique, on introduit progressivement 250 ml d'ammoniac liquide puis on ajoute 1,18 g du dérivé n° 4, la méthyl-4 benzyl-5 2-H 5-H pyrido (3', 4' : 4,5) pyrrolo (3,2-c) pyridone-1. L'ensemble étant maintenu sous agitation à -34°C, on ajoute du sodium en petits morceaux jusqu'à ce que la coloration bleue persiste plus de 30 minutes après la dernière addition. Après une heure supplémentaire à la température de reflux de l'ammoniac, le mélange est décoloré par addition de chlorure d'ammonium et abandonné pour laisser évaporer l'ammoniac. Le solide résiduel est repris dans environ 30 ml d'eau glacée, filtré puis recristallisé dans l'éthanol. On recueille 500 mg de microcristaux incolores.

Rendement : 56,4 p. 100 - F = $>$ 270°C.

Calc. pour $C_{11}H_9N_3O$, $H_2O$ : C, 60,82 ; H, 5,10 ; N, 19,35

Trouvé : C, 60,81 ; H, 5,11 ; N, 19,43

RMN $H_1$ $((CD_3)_2SO)$ ; $\delta$ : 2,29 (d, 3H, $CH_3$-4) ; 7,21 (d, 1H, H-3, $J_3$-$CH_3$-4 = 1Hz) ; 7,49 (q, 1H, H-6, $J_6$-$_7$ = 5,8 Hz, $J_{6-9}$ = 1 Hz) ; 8,38 (d, 1H, H-7), 9,26 (d, 1H, H-9) ; 11,10 (s, 1H, NH-5) ; 12 (s, 1H, NH-2)

Les dérivés qui viennent d'être décrits servent d'intermédiaires après chloration en position 1 dans la préparation des composés de formule A selon le schéma réactionnel suivant :

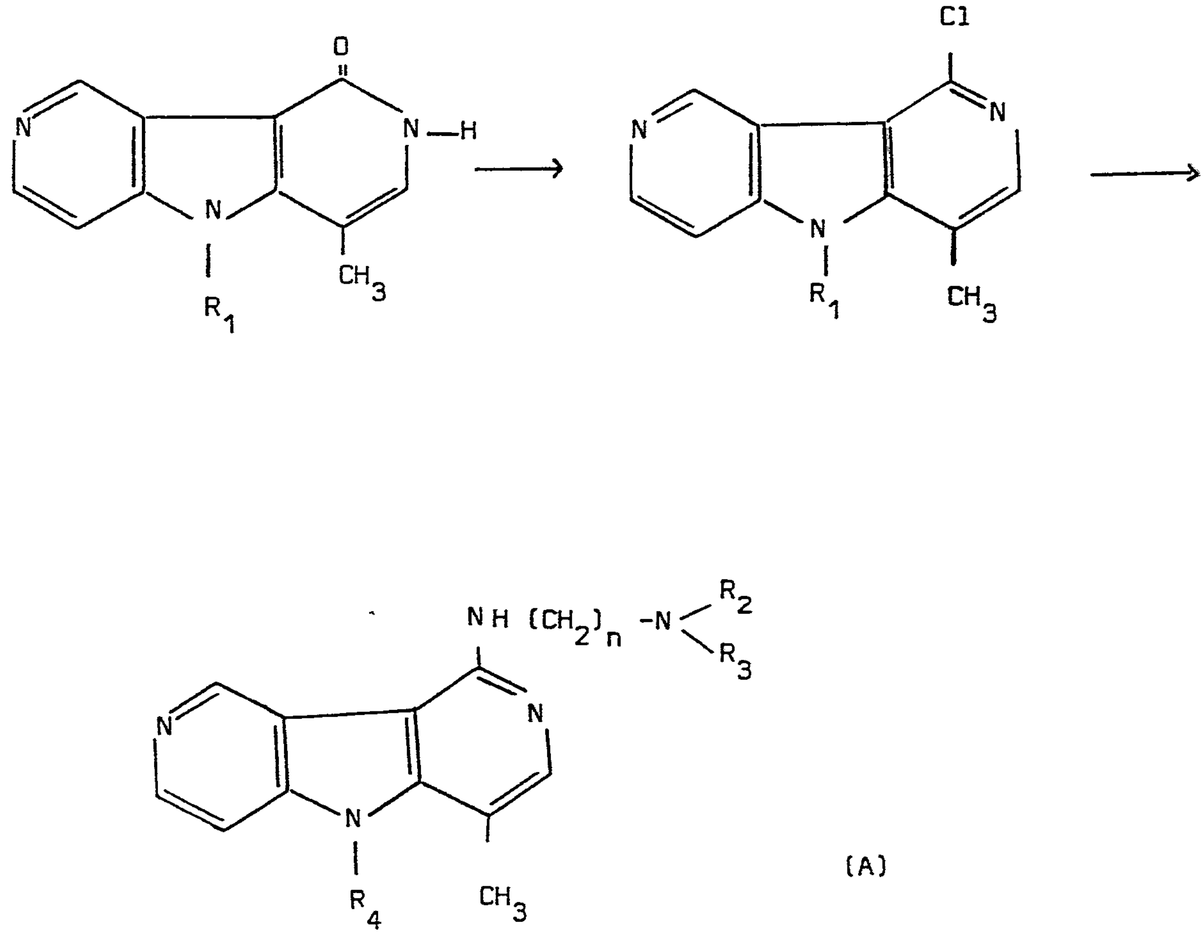

(A)

A titre illustratif du procédé de l'invention, on décrit ci-dessous la préparation de la (γ-diéthylamino) propylamino-1 diméthyl-4,5 5-H pyrido [ 3' , 4' : 4,5 ] pyrrolo [ 3,2-c ] pyridine (composé de formule A) à partir de la diméthyl-4,5 2-H 5-H pyrido [ 3', 4' : 4,5 ] pyrrolo [ 3,2-c ] pyridone-1 (dérivé N° 3 de formule I)

500 mg du dérivé N° 3 (2,5 mmoles) et 25 ml du dichlorure de l'acide phényl phosphonique sont chauffés au bain d'huile à 170°C sous agitation, pendant 68 heures, et l'excès de dichlorure est évaporé à 130°C sous 2 mm Hg. On ajoute 120 ml d'eau au résidu et le mélange est chauffé à l'ébullition puis filtré. L'insoluble est lavé avec 40 ml d'eau bouillante et le filtrat est alcalinisé à froid par addition d'ammoniaque.

Le précipité formé est essoré, séché et recristallisé dans le xylène pour fournir 450 mg de micro-cristaux incolores de la chloro-1 méthyl-4 5-H pyrido [ 3' , 4' : 4,5 ] pyrrolo [ 3,2-c ] pyridine .

Rendement : 82,3 p. 100 ; F = infusible à 270°C .

250 mg du composé (1,02 mmole) obtenu et 15 ml de diéthylamino propylamine sont chauffés sous agitation pendant 24 heures et l'excès d'amine est évaporé au bain-marie à 80° C sous 1 mm Hg de pression. Le résidu est repris dans 100 ml d'une solution d'hydroxyde de sodium N et extrait au chlorure de méthylène. Après évaporation, le produit est chromatographié sur colonne d'alumine en éluant avec le mélange chlorure de méthylène-éthanol 9/1.

Les fractions contenant le produit principal pur sont réunies et évaporées à sec. Le résidu est repris dans l'éthanol saturé par l'acide chlorhydrique et de noveau évaporé à sec. Le solide résiduel est lavé deux fois avec 10 ml d'acétone, cristallisé dans le n-butanol, essoré, lavé trois fois avec 10 ml d'acétone, repris dans 10 ml du même solvant, filtré et séché pour donner 340 mg de micro-cristaux incolores correspondant au trichlorhydrate trihydrate du composé recherché de formule A.

Ce composé suinte vers 130°C et se sublime à partir de 200°C.

Calc. pour $C_{19}H_{27}N_5$, 3 HCl, $3H_2O$ : C, 46,73 ; H, 7,37 ; N, 14,33 ; Cl, 21,80 .

Trouvé : C, 46,48 ; H, 7,81 ; N, 13,88 ; Cl, 22,08.

# REVENDICATIONS

1. Intermédiaires de synthèse de formule

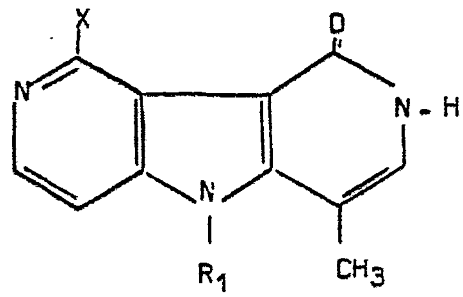

(I)

dans laquelle X représente l'hydrogène ou le chlore, $R_1$ représente l'hydrogène, un groupe alcoyle inférieur ou un groupe benzyle, ainsi que les formes tautomères lorsqu'elles existent.

2. Chloro-9 diméthyl-4,5 2-H 5-H pyrido (3', 4' : 4,5) pyrrolo (3,2-c) pyridone-1 ;

3. Chloro-9 méthyl-4 benzyl-5 2-H 5-H pyrido (3', 4' : 4,5) pyrrolo (3,2-c) pyridone-1 ;

4. Diméthyl-4,5 2-H 5-H pyrido (3', 4' : 4,5) pyrrolo (3,2-c) pyridone-1 ;

5. Méthyl-4 benzyl-5 2-H 5-H pyrido (3', 4' : 4,5) pyrrolo (3,2-c) pyridone-1

6. Méthyl-4 2-H 5-H pyrido (3', 4' : 4,5) pyrrolo (3,2-c) pyridone-1 ;

7. Procédé de préparation des composés de formule (I) caractérisé en ce que

a) on oxyde une (hydroxy-1 éthyl-1)-2 chloro-4 1-H pyrrolo (3,2-c) pyridine substituée en position 1, de formule

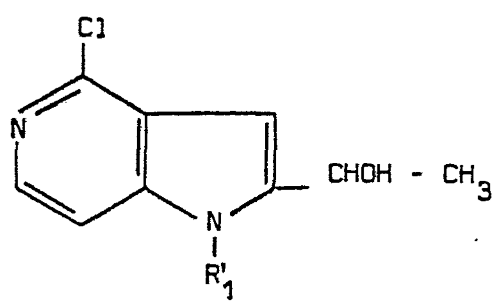

(II)

dans laquelle $R'_1$ est un groupement alcoyle inférieur ou benzyle en acétyl -2 pyrrolo-pyridine correspondante de formule

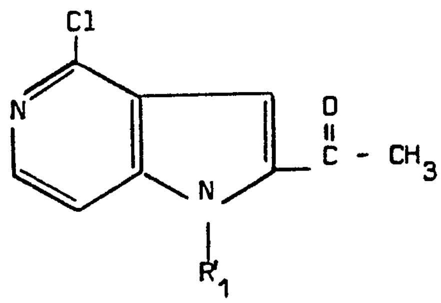

(III)

dans laquelle $R'_1$ est comme dans la formule II,

b) on transforme par une réaction de Wittig-Horner ce composé en (pyrrolo-pyridyl-2)-3 crotonate correspondant, de formule

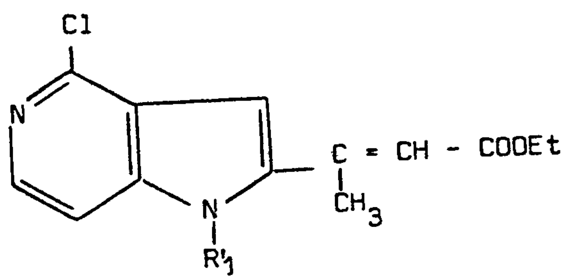

(IV)

dans laquelle $R'_1$ est comme dans la formule II,

c) on saponifie l'ester obtenu de formule IV pour obtenir l'acide correspondant de formule

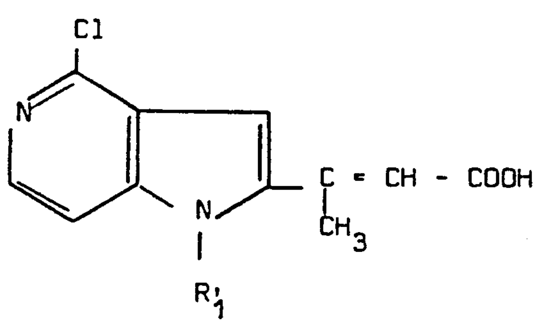

(V)

dans laquelle $R'_1$ est comme dans la formule II,

d) on transforme ce composé de formule V en azide correspondant de formule

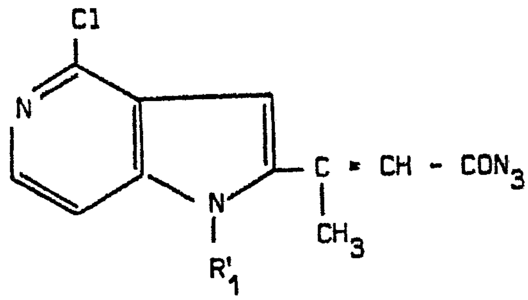

(VI)

dans laquelle $R'_1$ est comme dans la formule II,

e) on cyclise l'azide de formule VI pour obtenir le composé de formule I, X étant le chlore,

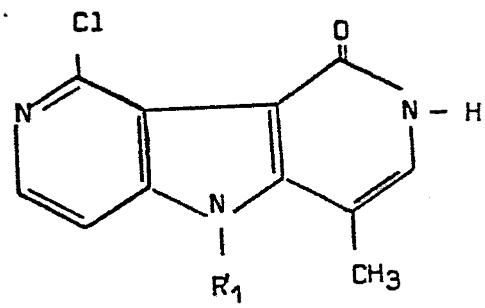

(I)

dans laquelle $R'_1$ représente un radical alcoyle inférieur ou benzyle, puis le cas échéant

f) par hydrogénation on obtient les composés de formule I, X représentant l'hydrogène,

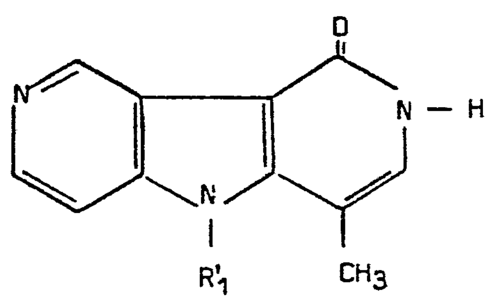

(I)

dans laquelle $R'_1$ représente un groupe alcoyle inférieur ou benzyle et, le cas échéant,

g) par débenzylation, on obtient le composé de formule (I) dans laquelle X et $R_1$ représentent l'hydrogène.

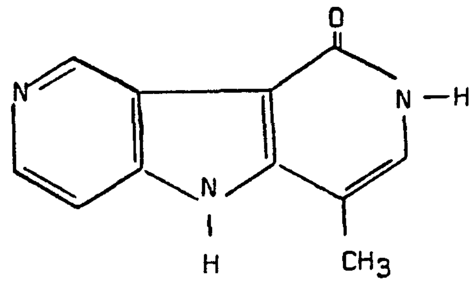

(I)

REVENDICATION pour l'état contractant AT

Procédé de préparation des composés de formule (I)

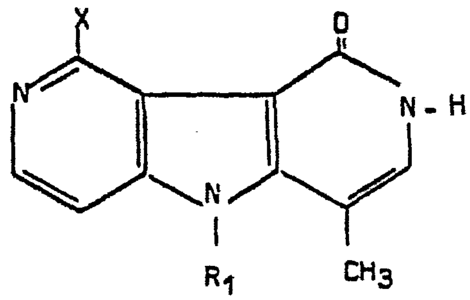

(I)

dans laquelle X représente l'hydrogène ou le chlore, $R_1$ représente l'hydrogène, un groupe alcoyle inférieur ou un groupe benzyle, ainsi que les formes tautomères lorsqu'elles existent, caractérisé en ce que

a) on oxyde une (hydroxy-1 éthyl-1)-2 chloro-4 1-H pyrrolo (3,2-c) pyridine substitué en position 1, de formule

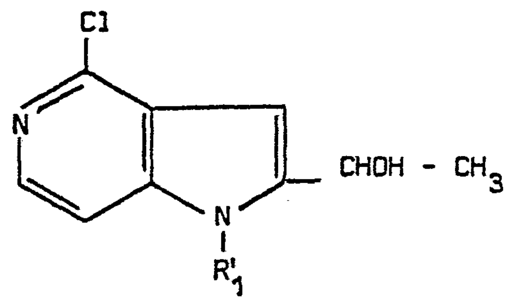

(II)

dans laquelle $R'_1$ est un groupement alcoyle inférieur ou benzyle en acétyl -2 pyrrolo-pyridine correspondante de formule

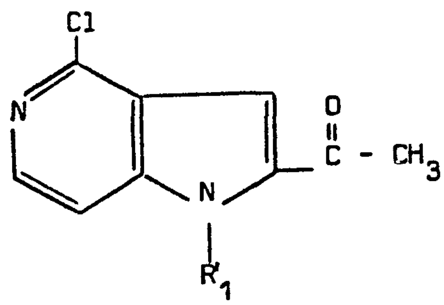

(III)

dans laquelle $R'_1$ est comme dans la formule II,

b) on transforme par une réaction de Wittig-Horner ce composé en (pyrrolo-pyridyl-2)-3 crotonate correspondant, de formule

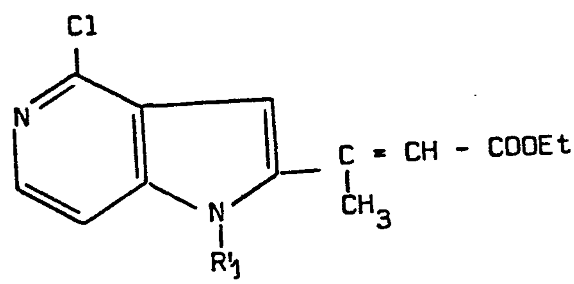

(IV)

dans laquelle $R'_1$ est comme dans la formule II,

c) on saponifie l'ester obtenu de formule IV pour obtenir l'acide correspondant de formule

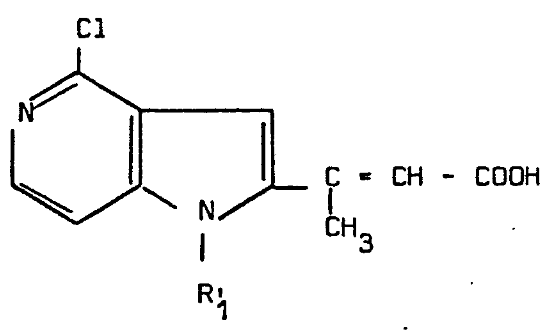

(V)

dans laquelle $R'_1$ est comme dans la formule II,

d) on transforme ce composé de formule V en azide correspondant de formule

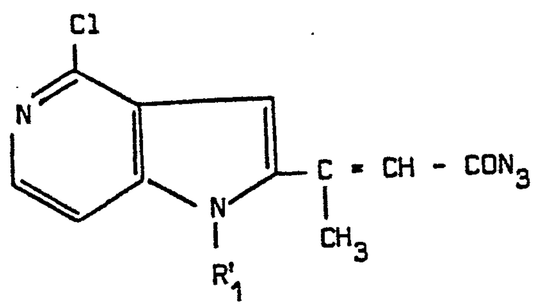

(VI)

dans laquelle $R'_1$ est comme dans la formule II,

e) on cyclise l'azide de formule VI pour obtenir le composé de formule I, X étant le chlore,

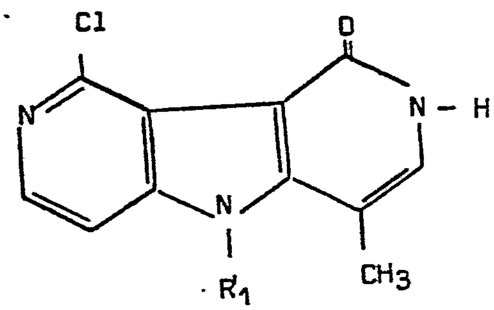

(I)

dans laquelle $R'_1$ représente un radical alcoyle inférieur ou benzyle, puis le cas échéant

f) par hydrogénation on obtient les composés de formule I, X représentant l'hydrogène,

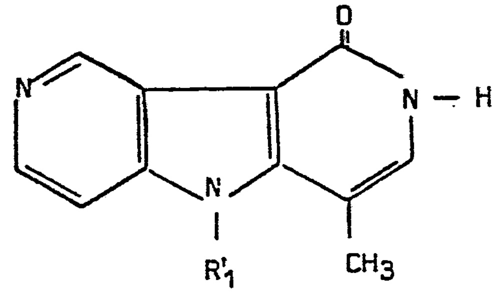

(I)

dans laquelle $R'_1$ représente un groupe alcoyle inférieur ou benzyle et, le cas échéant,

g) par débenzylation, on obtient le composé de formule (I) dans laquelle X et $R_1$ représentent l'hydrogène.

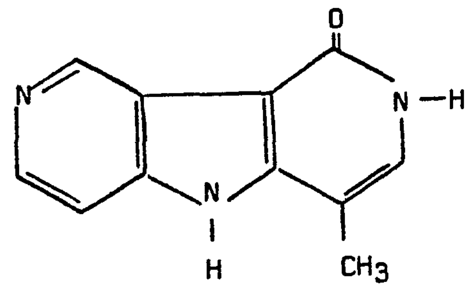

(I)

Office européen des brevets

# RAPPORT DE RECHERCHE EUROPEENNE

0199618

Numéro de la demande

EP 86 40 0603

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| | Néant<br>----- | | C 07 D 471/14 //<br>(C 07 D 471/14<br>C 07 D 221:00<br>C 07 D 221:00<br>C 07 D 209:00) |
| | | | **DOMAINES TECHNIQUES RECHERCHES (Int. Cl.4)** |
| | | | C 07 D 471/00 |

Le présent rapport de recherche a été établi pour toutes les revendications

| Lieu de la recherche | Date d'achèvement de la recherche | Examinateur |
|---|---|---|
| LA HAYE | 27-06-1986 | ALFARO I. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82